# EUROPEAN PATENT APPLICATION

(11) **EP 1 138 295 A1**
(43) Date of publication of application: **04.10.2001**
(21) Application number: 01100421.5
(22) Date of filing: 08.01.2001
(51) Int. Cl.: A61F 13/15

(54) **Flexible sanitary napkin**

(30) Priority: 15.03.2000 EP 00200935
(71) Applicant: THE PROCTER & GAMBLE COMPANY, Cincinnati, Ohio 45202 (US)
(72) Inventor: D'Alessio, Nicola, 65126 Pescara (IT); Dipilla, Mario, 65100 Pescara (IT); Kitzinger, Ulrike Dagmar Frauke, 65529 Waldems (DE); Pace, Anthony, 61462 Königstein (DE)
(74) Representative: Hirsch, Uwe Thomas M.H.

(57) **Abstract**

The present invention relates to menstrual absorbent articles, such as female sanitary napkins which are designed to be worn in and attached to a thong slips or G-string undergarment. In general absorbent articles for wearing with and attached to an undergarment should be designed to follow the shape of the undergarment where they are attached. For a thong slip this results in a sanitary napkin or panty liner having a wider front end and a narrower rear end. This design can also be described as triangular shape or trapezoidal shape. According to the present invention such thong shaped menstrual absorbent articles are provided in a construction in which the kind and amount of material, in particular for the absorbent core, and construction of the article, is selected such that the flexure resistance of the article remains below a critical value. In conjunction with the intimate wearing conditions achieved by the use of a thong slip this results in fitting of the sanitary napkin of the present invention in a very close proxinity to the wearer body contour providing for 'absorption at source' and little, if any space for liquid to escape. Articles constructed according to the present invention will display an absorbent and leakage performance in use, which surpasses even articles with a substantially higher amount of absorbent material.

## Description

### Field of the Invention

The present invention relates to menstrual absorbent articles, such as female sanitary napkins which are designed to be worn in and attached to a thong slips or G-string undergarment. In general absorbent articles for wearing with and attached to an undergarment should be designed to follow the shape of the undergarment where they are attached. For a thong slip this results in a sanitary napkin or panty liner having a wider front end and a narrower rear end. This design can also be described as triangular shape or trapezoidal shape. According to the present invention such thong shaped menstrual absorbent articles are provided in a construction in which the kind and amount of material, in particular for the absorbent core, and constuction of the article, is selected such that the flexure resistance of the article remains below a critical value. In conjunction with the intimate wearing conditions achieved by the use of a thong slip this results in fitting of the sanitary napkin of the present invention in a very close proxinity to the wearer body contour providing for 'absorption at source' and little, if any space for liquid to escape. Articles constructed according to the present invention will display an absorbent and leakage performance in use, which surpasses even articles with a substantially higher amount of absorbent material.

### Background of the Invention

In constructing sanitary napkins several considerations have generally been accepted. For example napkins for wearing with and attached to an undergarment should be designed to follow the shape of the undergarment where they are attached. This causes considerable manufacturing complexity since a strait sided napkin is much easier to make and hence less costly. However shaped sanitary napkins or panty liners have prevailed commercially due to their technical benefit of better fit to the undergarment and wearer. Recently a new undergarment style, thong slips or G-string undergarments have achieved increased in popularity and have raised the demand for respectively designed thong sanitary napkins and panty liners. Examples of such designs can be found in WO-A-97.39.713 (to Darby), WO-A-00.30.585 (to Drevik), or DE-A-295.13.548 (to Aurisch). Commercial products include e.g. Libresse String Sanitary Napkins ™ of SCA Hygiene Products of Sweden.

Another generally accepted desire for sanitary napkins or panty liners is that they shall be flexible and pliable for comfort by following the undergarment when the wearer moves. Improved flexibility was also considered to enhance the performance of absorbent articles since this would allow them to come closer to the vaginal orifice, i.e. the liquid source. This is e.g. described in EP-A-0.336.578 (to Osborn), EP-A-0.705.585 (to Querqui), EP-A-0.705.586 (to Querqui), or EP-A-0.705.584 (to Hirsch). On the other hand a limitation to flexibility was also realized already by Querqui and Hirsch and discussed already in US-A-4.217.901 (to Bradstreet).

However it has been considered that both these design/construction desires competed with the fundamental and functional need that the absorbent capacity of the article is sufficient to acquire and hold all the liquid emanating from the vaginal orifice. Napkins with the conventional dog-bone or hour glass shape have a center region where highest absorbency is needed. This center region has e.g. been provided with additional absorbent material relative to the front and rear regions.

Alternative designs included liquid transport constructions where the capacity outside the central area would also provide storage capacity for absorbed liquid by transport of the liquid inside a distribution layer within the absorbent core construction. Both attempts were successful and modern sanitary napkins often have an increased capacity in the central region and utilize transport construction elements for more efficient usage of the absorbent capacity outside the center. Similarly, in order to handle the differing flow quantities of menstrual liquid, sanitary napkins with higher amounts of absorbent material are available under labels such as "night", "super" or "extra" absorbent napkins.

However most articles still have not achieved the desired balance of absorbent capacity and flexibility/ liability in articles of conventional shape. For the new thong shape design no model of how to balance the absorbency, capacity, and comfort has been found hereto forth. The commercially available Libresse™ mentioned above addresses this balance in favor of absorbent capacity. Since in the hierarchy of needs functional requirements (i.e. sufficient absorbent capacity to prevent leakage during use) dominate the non-functional desires (i.e. comfort, aesthetic appearance) the Libresse™ string sanitary napkin is provided with a high capacity absorbent core using flash dried cellulose compounds. This in turn creates in the product a high degree of flexure resistance, causing potentially discomfort to the wearer. However in addition to this non-functional drawback an additional functional issue arises due to the stiffness the leakage prevention performance of these articles is not optimal. It is speculated that this results from gaps and misfits due to lack of intimate molding of the article to the user.

According to the present invention menstrual absorbent articles are provided with an absorbent structure, sandwiched between a liquid permeable topsheet and a liquid impermeable backsheet having a flexure resistance and pliability, which allows the articles to follow the natural shape and fit a thong undergarment will provide if worn without any absorbent article, namely intimately molded to the body of the wearer, in particular in the region of the vaginal orifice and the crease of the buttocks. This addresses the issues with existing products regarding comfort and at the same time solves the dilemma between a too low absorbent capacity and the need for reduced flexure resistance by a low material usage.

### Summary of the Invention

According to the present invention menstrual absorbent articles are provided with an absorbent core or structure, conventionally sandwiched between a liquid permeable topsheet and a liquid impermeable backsheet having a flexure resistance and pliability, as measured in accordance with a modified circular bend procedure defined below, in the range of 600 grams or less, preferably 550 grams to 200 grams, more preferably 500 grams to 300 grams, most preferably between 500 grams and 450 grams. The absorbent core is the structure within the napkins according to the present invention providing the main part of the flexure resistance. In order to limit the flexure resistance added to the flexure resistance of the absorbent core the ratio between the value of flexure resistance of the absorbent core to the value of the flexure resistance of the whole absorbent napkin is above 0.5, preferably above 0.55, more preferably above 0.6, and most preferably above 0.65.

In preferred embodiments of the present invention wings or side flaps for wrapping the side edges of the crotch portion of the undergarment, especially the narrow thong portion of the undergarment, are provided by portions of the topsheet and the backsheet extending beyond the periphery of the absorbent core. Such flaps are preferably provided with attachment means and in particular preferred embodiments they extend all the way to the rearward end of the menstrual article. Finally for masking of the article as such, but at least of the side flaps, but also for masking of components of the absorbent core, for masking of the liquid absorbed into the article, or for masking of residue liquid remaining on the topsheet side of the article coloration of the article or parts or portions of the article is particularly preferred. Especially dark coloration, such as black, dark red, dark green, or dark blue is also in line with fashion aspects of currently frequently used thong undergarments.

### Brief Description of the Drawing

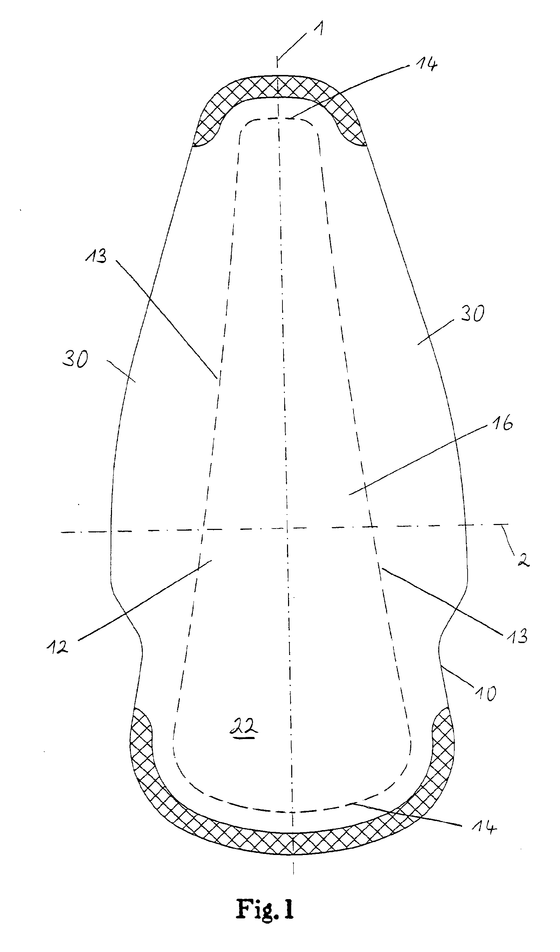

Fig 1. Is a plan view of a preferred absorbent article of the present invention with side flaps.

### Description of the Preferred Embodiments

As used herein, the term "longitudinal" refers to the direction of the central longitudinal axis referred to by reference numeral 1 in Fig 1. As used herein the term "transverse" refers to the direction of the transverse axis referred to by reference numeral 2 in Fig 1 and which is generally perpendicular to the central longitudinal axis.

As used herein, the term "non folded configuration" or "unfolded configuration" refers to the configuration in which the side flaps of the absorbent article are spread out in the same plane as the absorbent article as illustrated in Fig 1.

The preferred embodiment of the absorbent article of the present invention is shown in Fig 1. The absorbent article in Fig 1 is generally referred to by reference numeral 10. Absorbent article 10 comprises a central absorbent pad, which is generally referred to by reference numeral 12. The central absorbent pad 12 has longitudinal edges 13 generally oriented parallel to the central longitudinal axis 1, transverse ends 14 generally oriented parallel to the transverse axis 2, a body facing side 22 and an undergarment facing side. Extending from each longitudinal edge 13 of the central absorbent pad 12 are flaps 30.

The central absorbent pad 12 usually will comprise a liquid pervious topsheet, an absorbent core 16, and a liquid impervious backsheet. However other configurations and designs in which the topsheet or the backsheet or both are integral parts of the absorbent core, are simply not provided or wherein the absorbent core is an integral part of another element of the article are also considered useful in the context of the present invention. The central absorbent pad 12 and the absorbent core have a generally thong, trapezoidal or triangular shape having a wider front than rear portion which is especially shaped for use in modern undergarments of the G-string or string tanga type. This shape is referred to as thong shape both for describing undergarments or napkins in accordance with the present invention.

The liquid pervious topsheet, if present should be compliant, soft feeling and non-irritating to the user's skin. It can be made from any of the conventional materials for this type of use. Examples of suitable materials are woven and non-woven polyester, polypropylene, nylon and apertured films, preferably 3 dimensionally apertured films. The outer surface of the topsheet, preferably around the locations of intended liquid penetration may be treated with a compound, which renders the surface more hydrophilic allowing the liquid to penetrate the topsheet easily. Alternatively the outer surface of the topsheet, preferably between the locations of liquid penetration, may be treated with a compound which renders the surface more hydrophobic allowing liquid deposited there to migrate fast to locations where it can penetrate the topsheet easily, thus reducing liquid build up on the topsheet. The inner surface of the top sheet may be secured to the surface of the absorbent core 16 for example by the use of an adhesive or by other bonding means such as compression and/or thermo-bonding. This contacting relationship also results in liquid penetrating the topsheet easier.

The absorbent core 16, shown in its outline by a dashed line, is positioned between the topsheet and the backsheet and comprises a fluid absorbing material. Fluid absorbing materials may be natural or synthetic or combinations thereof. Examples of absorbent materials include cellulose fibers and super-absorbent gelling materials. Absorbent materials may be used either alone or in combination with other materials, both absorbent or not. The absorbent core can be provided as a single entity or comprise several layers.

The backsheet if present is impervious to liquids or retarding liquid penetration sufficient for the intended purpose of preventing fluid from the absorbent core migrating easily towards an underlying garment and soiling the garment or body of the user. The backsheet can be made from any of the conventional materials for this type of use. Suitable materials include embossed or nonembossed polyethylene, polypropylene, or other thermoplastic films or non-wovens . Especially breathable materials or multiple layer composites are preferred for use in the sanitary napkins according to the present invention.

For constructing sanitary napkins according to the present invention it is preferred that the topsheet and the backsheet extend beyond the periphery of the absorbent core and are attached to each other in the peripheral region around the absorbent core. Particularly beneficial is the crimping, indicated by hatched lines in figure 1, in the front and rear peripheral region, while in the peripheral region around the absorbent core adjacent the side flaps the attachment is achieved by adhesive combination. The peripheral region around the absorbent core, if present for joining the topsheet to the backsheet, in accordance with the present invention is part of the central absorbent pad (12). Beyond the central absorbent pad only side flaps for folding around the edge of the undergarment are optionally present. Depending on the detailed construction of the optional side flaps this folding can include the peripheral region formed by the topsheet and the backsheet.

Also preferably a portion of the undergarment facing side of the backsheet is coated with a panty-fastening adhesive. The adhesive is intended to fix the central absorbent pad to the crotch of the undergarment during use of the article. Any adhesive used in the art for such purpose can be used herein, with pressure sensitive adhesives being preferred. Before the sanitary napkin is placed in a thong undergarment the adhesive should be covered by a removable release liner to prevent the adhesive from drying out, being contaminated, or sticking to a surface other than those intended for that purpose. Silicone coated paper strips or polymeric filmstrips can provide such removable release liners. In particular preferred embodiments the release liner material also provides an individual wrapping of the thong sanitary napkin.

Preferred sanitary napkins according to the present invention further comprise flaps (30) which are folded about the edge of the undergarment's leg openings in order to provide additional protection from soiling the undergarment and, provided the flaps are equipped with an attachment means such as the panty fastening adhesive mentioned above or a mechanical attachment means on their garment facing surface, to support fixation of the article to the undergarment during use. The flaps (30) can be made of any suitable material. They can be integral extensions of one, some or all of the materials used to provide the central absorbent pad (12). Alternatively the flaps (30) can be constructed separately from the absorbent central pad (12). In this case they can be made from the same materials used already for the absorbent pad or form different materials such as those mentioned herein already. If provided separately the flaps are attached to the central absorbent pad (12) by any means conventionally used in combining absorbent articles, such as adhesives, thermo, or mechanical bonding. A key benefit of the integral construction is the ease of manufacture while the attached flaps provide benefits in material consumption and flexibility in material selection for the article manufacturer.

In an embodiment according to the present invention the flaps (30) comprise a flap topsheet and a flap backsheet. In a preferred embodiment the flap topsheet is integral with the topsheet. In another preferred embodiment the flap backsheet is also integral with the backsheet. In a preferred embodiment for use as a thong shaped sanitary napkin no absorbent material or only a very thin absorbent layer such as a tissue is provided as absorbent material in the flaps.

Flaps (30) can be essentially of any shape. As illustrated in Fig 1 the flaps (30) may have the same shape but they can also be provided asymmetrically with respect to the longitudinal axis(1) such that they do not overlap when folded in use. The flaps may have a different shape to achieve this purpose. Any differing shapes between the flaps may be used to ensure they do not overlap when folded around the thong undergarment. Of course flaps (30) can be identical in shape as shown in Fig. 1. The flaps (30) can extend along a small or a long portion of the central absorbent pad (12). It is preferred that the flaps be as long as practical so as to maximize the extent of undergarment side edge which is covered. In this respect it is possible and preferred that both flaps (30) extend all the way to the rear end of the absorbent article.

Especially string shaped undergarments and with them thong shaped sanitary napkins and panty liners have recently become fashionable in non-white coloring. It is therefore within the scope of the present invention to provide such napkins or panty liners with features suitable for the aesthetic, cosmetic and discretion/masking aspects triggering the use of string undergarments such as general fashion colors besides white, especially black, dark red, dark green or dark blue for all or some part of the article. In particular providing articles at least with flaps, which are colored so as to maintain the visual discretion achieved by the use of a thong napkin in a thong undergarment, by providing them in a color matching the color of the undergarment, is an especially appreciated article configuration. In another aspect to coloration the masking of the absorbent core - when it is unsoiled or soiled - but also of any residue liquid remaining on the topsheet by use of dark coloration of the topsheet and backsheet provides a visual benefit to the articles according to the present invention. In another aspect to ensure that the flaps are not visible during use they can be provided in a transparent or translucent design such replicating the color of the underlying material.

### Flexure resistance

A key aspect of the sanitary napkins according to the present invention is their flexure resistance behavior. Flexure resistance is a measurement which allows to quantify considerations of pliability, flexibility or stiffness, 'Schmiegsamkeit', and capability to follow and resemble the surface contour or topography of another structure.

The absorbent core provides the main component of the value of flexure resistance of the sanitary napkin. It is therefore essential that the absorbent core does not create a barrier to comfort and functionality of the thong sanitary napkin by having too high a flexure resistance. However the other materials and the construction of an absorbent article in general will increase flexure resistance as well. In particular the conventional layered construction of topsheet, core and backsheet, potentially with multiple layers in each of these components, where the layers are attached across their surface will increase flexure resistance. The skilled practitioner will understand that there is a positive correlation between the material quantity used in such constructions and the flexure resistance. Hence one way of influencing the flexure resistance is inclusion or removal of material.

But also the joining of the layers has a substantial effect on the flexure resistance of the final article. The lowest effect is to be expected by pure frictional attachment when the layers are simply laid on top of each other.

Obviously functional criteria, e.g. that the napkin must remain unified during all use conditions, will not allow such a frictional construction: Therefore at least a minimum of attachment between the layers is needed. This can be provided by yet additional material such as adhesive, or by mechanical entanglement such as crimping, or by thermal attachment such as welding or ultrasonic bonding. Also the amount of surface involved in the attachment has an influence in on the flexure resistance. E.g. creating a peripheral region attachment allows the layers between the peripheral region to slide over each other, while the same amount of attachment surface between layers but distributed over the whole surface will result in a substantially higher flexure resistance.

Naturally other aspects than flexure resistance need to be taken into account when constructing an article, such as stability of the construction (the article must remain intact at least during use), aesthetics of the attachment (e.g. hatched crimping or flower pattern crimping), cost of material and availability of processing facilities. Taking all this into account it has been found that the flexure resistance of the article as a whole needs to be limited to 600 or less gram but also above 200g, preferably above 300g, more preferably above 450g. In order to ensure that the other items besides the absorbent core increasing the flexure resistance are not over representing flexure resistance the ration of the value of flexure resistance of the core to flexure resistance of the whole napkin preferably has to be more than 0.5.

With the absorbent core being the dominating flexure resistance aspect the absorbent capacity is limited by the amount of material. Hence the performance of such articles would be expected to suffer, which however is not the case as will be shown below. In order to allow the maximum capacity to be provided, it is particularly beneficial if the absorbent core is provided with a pre-scoring in order to reduce its flexure resistance. Such pre-scoring is preferably parallel to and along its longitudinal centerline such that the garment facing surface of the absorbent core on the right and left side of the longitudinal centerline more readily fold towards each other. Scoring can be on the garment facing side or on the wearer facing side of the absorbent core. Preferred are longitudinally symmetrical scoring lines offset from the longitudinal centerline, especially in the center and front half of the thong sanitary napkin. In addition transverse scoring can also be provided to further increase flexure resistance.

Naturally any modification conventional in the art to the components or materials or construction of the absorbent articles according to the present invention, provided the inventive aspect of the low flexure resistance as included in the appended claims are preserved, will be at the discretion of the skilled practitioner and will not deviate from the present invention.

### Measurement method for flexure resistance:

This method is identical to the modified circular bend test procedure according to patent EP 336578. The sanitary napkin of the present invention has a low flexure-resistance. Thus, the sanitary napkin of the present invention is highly flexible and conforms very well to the various shapes of the female uro-genital region. The sanitary napkin of the present invention has a flexure-resistance of less than 6.0 grams.

The flexure-resistance of a sanitary napkin is measured by peak bending stiffness. Peak bending stiffness is determined by a test which is modeled after the ASTM D 4032-82 CIRCULAR BEND PROCEDURE, the procedure being considerably modified and performed as follows. The CIRCULAR BEND PROCEDURE is a simultaneous multi-directional deformation of a material in which one face of a specimen becomes concave and the other face becomes convex. The CIRCULAR BEND PROCEDURE gives a force value related to flexure-resistance, simultaneously averaging stiffness in all directions.

### APPARATUS:

The apparatus necessary for the CIRCULAR BEND PROCEDURE is a modified Circular Bend Stiffness Tester, having the following parts:

A smooth-polished steel plate platform which is 102.0 x 102.0 x 6.35 millimeters having an 18.75 millimeter diameter orifice. The lap edge of the orifice should be at a 45 degree angle to a depth of 4.75 millimeters.
A plunger having an overall length of 72.2 millimeters, a diameter of 6.25 millimeters, a ball nose having a radius of 2.97 millimeters and a needle-point extending 0.88 millimeter therefrom having a 0.33 millimeter base diameter and a point having a radius of less than 0.5 millimeter, the plunger being mounted concentric with the orifice and having equal clearance on all sides. Note that the needle-point is merely to prevent lateral movement of the test specimen during testing. Therefore, if the needle-point significantly adversely affects the test specimen (for example, punctures an inflatable structure), than the needle-point should not be used. The bottom of the plunger should be set well above the top of the orifice plate. From this position, the downward stroke of the ball nose is to the exact bottom of the plate orifice.
A force-measurement gauge and more specifically an Instron inverted compression load cell. The load cell has a load range of from 0.0 to 1000 grams. Of course force measurements are not identical to mass measurements. However, the gauge is set such that it displays a value of mass excerting the equivalent force under gravity.
An actuator, and more specifically the Instron Model No. 6021 having an inverted compression load cell. The Instron 6021 is made by the Instron Engineering Corporation, Canton, Massachusetts.

### NUMBER AND PREPARATION OF SPECIMENS

In order to perform the procedure for this test, as explained below, five representative sanitary napkins are necessary. From one of the five napkins to be tested, some number "Y" of 37.5 x 37.5 millimeter test specimens are cut. Specimens should be representative of the center of the napkin, or the region of highest apparent stiffness and not include portions in which e.g. a topsheet is joined directly to a backsheet. The reason that these specimens are not tested is due to the realization that prior art napkins exist in which a topsheet is joined to a barrier sheet beyond the edges of an absorbent core in the periphery of the napkin, such portions of which are highly flexible. However, the present invention is concerned with the overall flexibility of the sanitary napkin and not merely the peripheral portions thereof and, therefore, the flexibility of the present invention is more concerned with the flexibility of the significant absorbent portions of the sanitary napkin. If any of these significant absorbent portions of the sanitary napkin meet the parameters of this test, then the sanitary napkin satisfies the test. Therefore, a number of different specimens should be tested from each sanitary napkin. For the purpose of the present invention, the structurally least flexible portion of the sanitary napkin should be tested. The test specimens should not be folded or bent by the test person, and the handling of specimens must be kept to a minimum and to the edges to avoid affecting flexural-resistance properties. From the four remaining sanitary napkins, an equal number "Y" of 37.5 x 37.5 millimeter specimens, identical to the specimens cut from the first napkin, are cut. Thus, the test person should have "Y" number of sets of five identical specimens.

The same sample preparation is done for the absorbent core, prior to installation of such core material into the sanitary napkin. Alternatively, or in order to evaluate existing sanitary napkins the absorbent core should be carefully removed between the topsheet and the backsheet.

### PROCEDURE

The procedure for the CIRCULAR BEND PROCEDURE is as follows. The specimens are conditioned by leaving them in a room that is 21 +/- 1 DEG C and 50 +/- 2% relative humidity for a period of two hours. The test plate is leveled. The plunger speed is set at 50.0 centimeters per minute per full stroke length. A specimen is centered on the orifice platform below the plunger such that the body surface of the specimen is facing the plunger and the garment surface of the specimen is facing the platform. The indicator zero is checked and adjusted, if necessary. The plunger is actuated. Touching the specimen during the testing should be avoided. The maximum force reading to the nearest gram is recorded. The above steps are repeated until all five of the identical specimens have been tested.

### CALCULATIONS

The peak bending stiffness for each specimen is the maximum force reading for that specimen. Remember that "Y" number of sets of five identical specimens were cut. Each set of five identical specimens is tested and the five values received for that set are averaged. Thus, the test person now has an average value for each of the "Y" sets tested. Remember, if any of the significantly absorbent portions of the sanitary napkin have the requisite flexure-resistance, then the napkin satisfies the parameters of this test. Therefore, the flexure-resistance for a particularly designed sanitary napkin is the greatest of these average peak-bending stiffness.

### Experimental core napkin flexure resistance data comparison

The following results were obtained with the above mentioned modified circular bend test procedure. The results are in grams. The products tested are:
- a commercially available thong product, Libresse™ String Sanitary Napkins with side flaps
- a commercially available sanitary napkin with side flaps for heavy loading conditions, Always™ Night Ultra, available from The Procter and Gamble Company, e.g. in Germany
- 4 experimental thong sanitary test napkins. All 4 were in the shape as shown in Figure 1, The topsheet was the same as in the commercially available Always™ product, an apertured formed film, available from The Procter & Gamble Company under the trade name Dryweave. The backsheet was the same polyethylene film as used in the commercially available Always™, having a thickness of 20 micrometer. Both the topsheet and the backsheet extended beyond the absorbent core as shown in Figure 1 forming side flaps for folding around the edge of the thong undergarment. The backsheet was provided with pressure sensitive adhesive in 3 regions, one each in the side flaps and one in the central region. The adhesive was the same as that on the commercially available Always™. The construction of the thong test napkins further included a fine, low basis weight web of adhesive between the topsheet and the absorbent core and between the core and the backsheet. A peripheral region around the thong test napkins was also provided to encase the absorbent core. The absorbent core in all of the products was provided by a MBAL core construction. MBAL is a fibrous material comprising cellulose fibers and bi-component (polypropylene shaft with ethylene coated on the outside) thermo-bondable fibers. In this fiber matrix super-absorbent particles are dispersed homogeneously. The MBAL material is available in different basis weights, where primarily the quantity of super-absorbent particles per square area changes and with it the absorbent capacity of the structure. The MBAL material is unified by use of thermal bonding and latex bonding on its surface. In 2 of the 4 thong test napkins 5 creasing lines were introduced running in longitudinal direction, substantially parallel and symmetrically to the longitudinal centerline. Also in 2 each, of the 4 thong test napkins different absorbent core capacities by use of different basis weights were used.

**Table 1**

| Product | Libresse String Sanitary Napkins | Always Night Ultra | Thong test napkin with MBAL 300 g/m² | Thong test napkin with MBAL 300 g/m² + 5 length wise flex lines | Thong test napkin with MBAL 180 g/m² | Thong test napkin with MBAL 180 g/m²+ 5 length wise flex lines |
|---|---|---|---|---|---|---|
| Super-absorbent particle basis weight (g/m²) | 0 | 118 | 105 | 105 | 31 | 31 |
| ^{*}Dunk Capacity (g absorbed/g-dry-weight) | 36 | 40 | 46 | 46 | 28 | 28 |
| Flexure resistance of the core alone | ^{**}Not measurab le | 357 g | 846 g | 489 g | 520 g | 336 g |
| Flexure resistance of the sanitary napkin | 3467 g | 705 g | NA | 877 g | NA | 489 g |
| Leakage diary test ^{***}(%chang es with leakage) | 60 | 40 | NA | 60 | NA | 45 |
| Flexure resistance ratio of core to napkin | NA | 0.507 | NA | 0.558 | NA | 0.687 |

| | | | | | | |
|---|---|---|---|---|---|---|
| **Remarks:** ^{*}- Dunk Capacity expresses a theoretical capacity of an absorbent structure when it is allowed to absorb de-ionized water without confining pressure. | | | | | | |
| ^{**}- A value of 1122 grams was obtained when setting the plunger speed to 1 cm/minute in the flexure resistance test. With an increase to 50 cm/minute the Libress™ core material was too brittle to be measurable. | | | | | | |
| ^{***}- In a Leakage diary test menstruating women use a sanitary napkin during menstrual cycle and return their undergarment with used article. The leakage performance is evaluated by a panel of graders who decided for each change whether the undergarment was soiled and calculate as a result the percentile of changes with leakage. | | | | | | |

As can be seen from Table 1 and as common and conventional expectation would have been the high capacity thong test napkins should have provided a better performance than the lower capacity thong napkins and possibly somewhat similar to the commercially available Always™ product. However the leakage performance is rather similar to the Libress™ product. In fact the two thong napkins with high absorbent capacity gave similar leakage diary results, but unexpectedly worse than the lower capacity, higher flexibility thong test napkin. When reducing capacity, and thereby reducing flexure resistance the performance of the article improves contrary to an expectation based on the capacity available for absorption.

## Claims

1. Disposable menstrual sanitary napkin having a wearer facing surface and a garment facing surface, and comprising
preferably a topsheet providing said wearer facing surface of said napkin and
preferably a backsheet providing said garment facing surface of said napkin, and
an absorbent core comprising an absorbent material sandwiched between said wearer facing surface and said garment facing surface, and
said napkin being shaped for use in combination with a thong undergarment, said absorbent core having a thong shape, and
said napkin having a flexure resistance, as measured in accordance with a modified circular bend procedure defined in the specification, in the range of 600 grams or less, preferably 550 grams to 200 grams, more preferably 500 grams to 300 grams, most preferably between 500 grams and 450 grams.

2. Sanitary napkin according to claim 1 **characterized in that** the ratio between the value of flexure resistance of said core to the value of said flexure resistance of said napkin is above 0.5, preferably above 0.55, more preferably above 0.6, and most preferably above 0.65.

3. Sanitary napkin according to any of the preceding claims **characterized in that** said topsheet or said backsheet or both said topsheet and said backsheet extend beyond at least part of the periphery of said absorbent core and form side flaps for folding about the crotch edge of a thong undergarment, preferably said topsheet and said backsheet extend beyond the whole periphery of said absorbent core and are sealed to each other to encase said absorbent core

4. Sanitary napkin according to any of the preceding claims **characterized in that** it comprises colored parts or regions.

5. Sanitary napkin according to any of the preceding claims **characterized in that** the thickness of said napkin is less than 5 mm, preferably less than 3.5 mm.

6. Sanitary napkin according to any of the preceding claims **characterized in that** said core comprises super absorbent gelling material.

7. Sanitary napkin according to claim 6 **characterized in that** said super absorbent gelling material is fibrous.
